# EUROPEAN PATENT SPECIFICATION

(11) **EP 1 658 099 B1**
(45) Date of publication and mention of the grant of the patent: **10.09.2008**
(21) Application number: 04777606.7
(22) Date of filing: 06.07.2004
(51) Int. Cl.: A61K 51/04

(54) **COMPOUNDS AND KITS FOR PREPARING IMAGING AGENTS AND METHODS OF IMAGING**
VERBINDUNGEN UND KITS ZUR HERSTELLUNG VON BILDGEBENDEN MITTELN UND BILDGEBUNGSVERFAHREN
COMPOSES ET KITS DE PREPARATION D'AGENTS D'IMAGERIE ET PROCEDES D'IMAGERIE

(30) Priority: 26.08.2003 US 647979
(43) Date of publication of application: 24.05.2006
(73) Proprietor: GENERAL ELECTRIC COMPANY, Schenectady, NY 12345 (US)
(72) Inventor: JOHNSON, Bruce, Fletcher, Scotia, NY 12302-2423 (US); SICLOVAN, Tiberiu, Mircea, Rexford, NY 12148 (US)
(74) Representative: Hammett, Audrey Grace Campbell
(86) International application number: PCT/US2004/021600
(87) International publication number: WO 2005/023317

(56) References cited:
- WO-A-02/085903
- WO-A-03/002157
- WO-A-20/04056399
- US-A- 4 659 517
- US-A- 4 775 759
- US-A- 5 264 570

## Description

### BACKGROUND OF THE INVENTION

This disclosure relates to methods and compositions for producing labeled targeting molecules suitable for medical imaging, such as, for example, positron emission tomography.

Positron emission tomography (PET) is a high resolution, non-invasive, imaging technique for the visualization of human disease. In PET, 511 keV gamma photons produced during positron annihilation decay are detected. In the clinical setting, fluorine-18 (¹⁸F) is one of the most widely used positron-emitting nuclides. ¹⁸F is most conveniently produced by cyclotrons in the form of ¹⁸F fluoride in an aqueous solution. The two-hour half-life of ¹⁸F makes it desirable for imaging; however, it places special demands on the synthesis/purification protocol used to convert the ¹⁸F fluoride into the desired radiopharmaceutical. The overall protocol must be rapid to minimize the loss of ¹⁸F from radioactive decay. Furthermore, the synthesis should be robust and simple so that it can be readily automated. Automation is desirable to minimize exposure of laboratory staff to radioactivity and to enable clinical implementation.

A common approach for the synthesis of ¹⁸F-labeled radiopharmaceuticals involves treatment of the ¹⁸F fluoride with a large excess of the pharmaceutical precursor activated by an electrophilic leaving group; the trifluoromethanesulfonate (triflate) group is commonly used in the PET community as the electrophilic leaving group. The large excess of precursor is used to ensure a high yield of labeled product based on starting ¹⁸F fluoride at reasonable reaction rates. The ¹⁸F fluoride displaces the electrophilic leaving group to create the desired labeled compound in a reaction known as nucleophilic displacement. Subsequent reaction(s) may be required to produce the desired ¹⁸F labeled radiopharmaceutical, although a prime goal of a PET radiochemical synthesis is to minimize the number of steps and duration of each step after incorporation of the label.

Following the nucleophilic displacement reaction, the crude reaction mixture consists of a small amount of the desired ¹⁸F-labeled compound and a large amount of unreacted precursor. It is often desirable or necessary to purify the desired ¹⁸F-labeled product from unreacted precursor and any by-products that have formed. In the case of trifluoromethanesulfonate, the desired ¹⁸F-labeled product often does not behave dramatically different from the unlabeled precursor. Thus a difficult time-consuming separation on a high-pressure liquid chromatograph (HPLC) may be required. This lengthens overall synthesis time (which results in lower yield of radioactive product), presents challenges for robust automation, limits the effective timeframe to conduct PET imaging and ultimately hinders the development of new PET radiopharmaceuticals.

US 5 264 570 discloses 1,3,4,6-tetra-O-acetyl-2-O-trifluoromethanesulfonyl-2-deoxy-beta-D-mannopyranose in a method of preparation of [¹⁸F]FDG in a reaction with [¹⁸F]fluoride.

US 4 775 759 describes a prodrug for the manufacture of ¹⁸F-opioid ligands.

US 4 659 517 discloses ¹²⁵I labelled estradiols for estrogen receptor assays by NMR, and methods for their preparation.

WO03/002157 relates to solid-phase nucleophilic fluorination of targeting moieties such as FDG, FDOPA, FLT, and FDDNP.

There is a need, therefore, for a simple, efficient method for incorporating the ¹⁸F radionuclide into targeting molecules to allow the use of such targeting molecules in routine clinical positron emission tomography.

### BRIEF DESCRIPTION OF THE INVENTION

Compounds that include a targeting moiety bound to a regioselective leaving group are described herein. The leaving group provides a site for regioselective substitution of a detectable species, resulting in the production of an imaging agent that is easily isolated from by-products derived from the leaving group. In certain embodiments, the imaging agent is isolated from by-products derived from the leaving group based on differences in the chemical attributes (e.g., net charge or polarity) of the molecules. In other embodiments, the imaging agent is isolated from by-products derived from the leaving group based on differences in the physical attributes of the molecules.

In a particularly useful embodiment of the latter sort, compounds that include a targeting moiety bound to a solid support via the leaving group are described herein. The leaving group provides a site for regioselective substitution of a detectable species, resulting in the release of an imaging agent from the solid support.

Methods of producing an imaging agent in accordance with this disclosure include the steps of providing a compound that includes a targeting moiety bound to a support via a linker group that contains a site for regioselective substitution of a detectable species, contacting compound with a solution containing the detectable species, and recovering the imaging agent.

In another aspect, a kit is described which includes a first container having therein a compound that includes a targeting moiety bound to a support via a leaving group that contains a site for regioselective substitution of a detectable species and a second container having therein a solution containing the detectable species.

### BRIEF DESCRIPTION OF THE DRAWINGS

Figure 1 shows a kit in accordance with one embodiment of the present disclosure.
Figure 2 shows a kit in accordance with another embodiment of the present disclosure.

### DETAILED DESCRIPTION OF THE INVENTION

Compounds in accordance with the present disclosure include a targeting moiety bound to a regioselective leaving group. The leaving group provides a site for regioselective substitution of a detectable species, resulting in the production of an imaging agent that is easily isolated from by-products derived from the leaving group. As used herein, the term "regioselective" means that one specific site on the molecule is preferentially reactive. Preferential reactivity can be as a result of steric hindrance, electrostatic interactions (repulsions or attractions) or a combination thereof. The term "imaging agent" refers to a targeting molecule having a detectable species associated (e.g., through covalent binding or chelation) therewith.

As used herein the term "targeting moiety" refers to any molecule which due to its chemical or physical attributes will tend to accumulate preferentially at particular sites when administered to a subject, such as, for example a human. The targeting moiety may be synthetic, semi-synthetic, or naturally occurring. Materials or substances which may serve as targeting moieties include, for example, proteins, including antibodies, glycoproteins and lectins, peptides, polypeptides, saccharides, including mono-and polysaccharides, vitamins, steroids, steroid analogs, hormones, cofactors, and genetic material, including nucleosides, nucleotides and polynucleotides. The accumulation can be provided by any process, such as, for example, by binding of the targeting moiety to a cell surface receptor or by preferential metabolism of the targeting moiety or by passage of the targeting moiety through openings present in certain tissue (e.g., diseased tissue) but not in all tissue.

Suitable targeting moieties include, but are not limited to, 2-deoxy-2-fluoro-D-glucose (FDG); 6-fluoro-L-DOPA (F-DOPA); 6-fluoro-L-meta-tyrosine (6-FMT); 9-[4-fluoro-3-(hydroxymethyl)butyl]guanine (FHBG); 3'-deoxy-3'-fluoro-thymidine (FLT); 2-methyl-2-fluoromethyl glycine; benzimidazole, benzothiazole, benzoxazole and thiovlavin T analogs such as those described in WO 02/085903.

In particularly useful embodiments, the targeting moiety is one that binds to soluble beta-amyloid, and can be a small molecule, peptide, protein, enzyme, dendrimer, polymer, antibody or antibody fragment. Antibodies specific for soluble beta-amyloid can be prepared against a suitable antigen or hapten comprising the desired target epitope, such as the junction region consisting of amino acid residues 13-26 and/or the carboxy terminus consisting of amino acid residues 33-42 of beta-amyloid. One suitable antibody to soluble beta-amyloid is disclosed in Kayed, et al., Science, vol. 300, page 486, April 18, 2003.

The regioselective leaving group is an organic moiety that contains a regioselective site at which substitution of a detectable species can take place and which, upon removal from the targeting moiety, can be easily separated from the resulting imaging agent. The exact nature of the reactive site on the leaving group will depend on the specific detectable species being employed. Thus, for example, the regioselective leaving group can include a sulfonate group as a site at which nucleophilic substitution by a halide (e.g., ¹⁸F) can take place. Other regioselective leaving groups which may be utilized with detectable species include N-alkyl-2-mercaptothiazolinium-2-yl; 1-alkyl-2-mercapto-pyrimidinium-2-yl; 1,4-dialkyl-2-mercaptopyrimidinium-2-yl; 5-alkyl-1,3-dimethyl-2-mercapto-benzimidazolium-2-yl; 5-alkoxy-1,3-dimethyl-2-mercapto-benzimidazolium-2-yl; 5-alkoxy-2-mercapto-3-methyl-benzothiazolium-2-yl; 5-alkoxy-2-mercapto-3-methylbenzoxazolium-2-yl; 3-alkyl-2-mercapto-1-methyl-imidazolium-2-yl; 4-aryl-2-mercapto-3-methylthiazolium-2-yl. Particularly useful leaving groups exhibit a rate of substitution comparable to that of a triflate leaving group. The rate at which the leaving group is substituted with a detectable species can be determined using any method known to those skilled in the art. Such methods include gas chromatography/mass spectrometry (GC-MS) and liquid chromatography/mass spectrometry (LC-MS). Quantitative analysis of the substituted product vs. time yields rate constant values for each pair of leaving group-detectable species.

In some embodiments, the precursors include 2-mercapto N-alkyl pyrimidines, 2-mercapto-5-alkoxy-N-alkyl benzothiazoles and benzimidazoles, and 2-mercapto-5-nitro-N-alkyl benzothiazoles and benzimidazoles, which are commercially available from Lancaster Research Chemicals and Aldrich Chemical Co.

The regioselective leaving group also contains structures which facilitate separation of the imaging agent from any by-products derived from the leaving group. The selection of the specific structure that facilitates separation will depend on a number of factors, including the characteristics of the imaging agent to be produced. In certain embodiments, the chemical characteristics of the leaving group facilitate separation of the imaging agent from any by-products derived from the leaving group. For example, where the imaging agent carries no charge, the leaving group can include a positively or negatively charged portion to facilitate separation (e.g., via an ion exchange resin). Alternatively, the leaving group can include a polar group to facilitate separation. Where the imaging agent produced contains a charge or a polar group, the leaving group should be designed in a manner that provides charge-neutral by-products. Separation of labeled product from precursor can thus be accomplished by passage of the reaction mixture through a short plug solid-phase media such as silica gel or ion exchange resin with an appropriate solvent. In particularly useful embodiments, separation results in removal of greater than 99% of precursor present after nucleophilic displacement using this simplified purification process.

Suitable leaving groups are selected from :
(i) Groups of the formula: 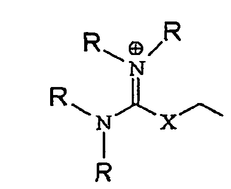 where X= S or O and R can be the same or different at each occurrence and is selected from C₁ to C₂₀ alkyl groups;
(ii) pyridinium and pyrimidinium salts, such as: 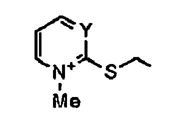 where Y is N or CH;
(iii) benzoxazolium/benzothiazoliun salts such as: 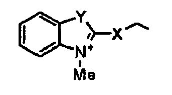 where when X is S, then Y is O or S, and where when X is O, then Y is S;
(iv) groups of the formula: 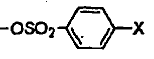 where X is selected from C₄ to C₁₀ alkylene, -CN, -N⁺(CH₃)₃, or -(Q)ₙOCH₃ where Q is C₂ to C₆ alkoxy and n = 1 to 6;
(v) groups of the formula *:* 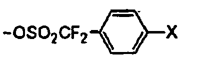 where X is selected from C₄ to C₁₀ alkylene, -CN, -N⁺(CH₃)₃, or-(Q)ₙOCH₃ where Q is C₂ to C₆ alkoxy and n = 1 to 6;
(vi) groups of the formula:

   -OSO₂CF₂·CH₂X
where X is selected from C₄ to C₁₀ alkylene, -CN, -N⁺(CH₃)₃, or-(Q)ₙOCH₃ where Q is C₂ to C₆ alkoxy and n = 1 to 6. In particularly useful embodiments of the last three exemplary groups, X is selected from -(CH₂)₅CH₃, -(OCH₂CH₂)₂OCH₃, -CN or - N⁺(CH₃)₃.

Compounds from which the foregoing groups can be derived are commercially available, e.g., from Lancaster Research Chemicals and Aldrich Chemical Co.

In other embodiments, the physical characteristics of the compound containing the leaving group facilitate separation of the imaging agent from any by-products derived from the leaving group. For example, the leaving group can be bound to a solid-phase support, such as, for example, a polymer bead of the type known to those skilled in the art of solid phase synthesis. The solid-phase support will typically be comprised of small porous beads or particles in the form of a resin or gel. Numerous materials are suitable as solid-phase supports for the presently contemplated synthesis. In general, such supports should provide good mass transfer in and out of their pores, be chemically inert, be minimally affected by reagents and solvents, and allow derivatization. Preferred solid-phase materials include polystyrene derivatives, controlled pore glass, aluminum oxide beads, and silica beads. Suitable compounds of this type include, but are not limited to: 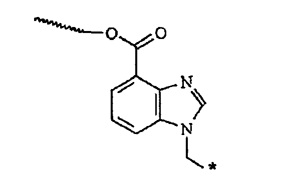 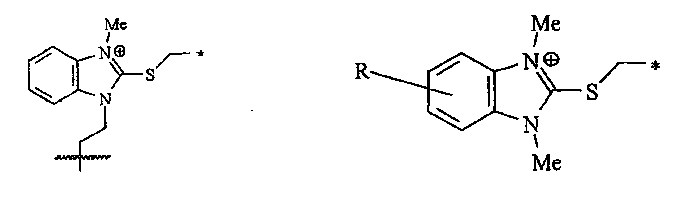 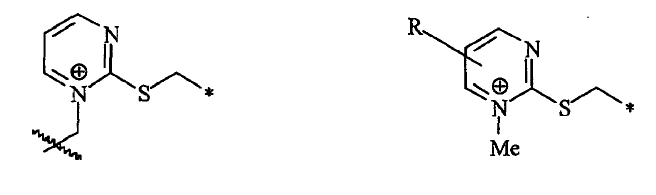 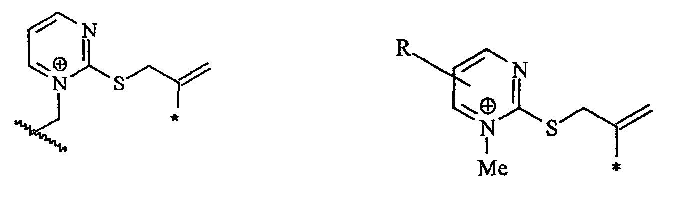 where * indicates the site at which the targeting moiety is located and R is a substituent which may also be used as a linker to the polymeric support. Suitable R groups include methyl (n-alkyl), alkoxy, and nitro. These materials are known to those skilled in the art and are commercially available.

The leaving group-targeting agent precursors can be prepared by a conventional alkylation procedure, readily apparent to one skilled in the art. In one embodiment, the alkylation procedure for producing these precursors involves the following steps: combining a starting material such as an imidazole, a pyrimidine or a benzimidazole with dichloromethane and then adding methyl triflate while stirring. The reaction is conducted under anhydrous conditions as water, which is also a nucleophile, can displace the imaging agent prematurely. In such a case an onium salt is produced in solution, to which a detectable species may then be added.

The detectable species can be any chemical entity that emits a detectable signal suitable for in vivo diagnostic imaging by positron emission tomography ("PET").

Such detectable species include, but are not limited to, "C, ¹⁸F, ¹²³I, and ¹²⁵I. Protocols for the synthesis of radiolabeled compounds are described in Tubis and Wolf, Eds., "Radiopharmacy", Wiley-Interscience, New York (1976); Wolf, et al., "Synthesis of Radiopharmaceuticals and Labeled Compounds Using Short-Lived Isotopes", in Radiopharmaceuticals and Labeled Compounds, Vol 1., pp. 345-381 (1973).

The detectable species can be reacted with the regioselective group using techniques known to those skilled in the art. Where the detectable species is ¹⁸F, a typical synthesis involves first activating the ¹⁸F through "activating" agents such as KRYPTOFIX^{™} (also called K2.2.2), a trademark used in connection with the compound 4,7,13,16,21,24-hexaoxo-1,10-diazabicyclo-[8.8.8]-hexacosane, so as to make it more reactive. In some publications, they are called "phase transfer agents". The radionuclide is produced beforehand, generally by irradiation of ¹⁸O enriched water with a proton beam originating from a particle accelerator, as F- (for instance H¹⁸F, in an aqueous solution). Next, the fluorinating agent, made totally anhydrous by additions of acetonitrile (CH₃CN) and dry evaporations, is combined with a compound of the present disclosure solubilized in acetonitrile. A substitution reaction then occurs, where the regioselective group on the leaving group of the present compound is attacked by the fluorinating agent resulting in production of the imaging agent and a byproduct. Similarly, one can introduce I and Br radioisotopes as detectable species. Additionally, where the detectable species is "C, nucleophiles such as methyllithium and methylmagnesiumbromide can be used. A simple separation (e.g., passage of the reaction mixture through an ion exchange column) can then be performed to isolate the imaging agent. It should of course be understood that where the leaving group is bound to a solid support, no separation step is required.

Once isolated, the imaging agent can be formulated into a composition comprising a pharmaceutical carrier and administered to a patient. A pharmaceutical carrier can be any compatible, non-toxic substance suitable for delivery of the labeled compound to the patient, and can include sterile water, alcohol, fats, waxes, proteins, and inert solids. Pharmaceutically acceptable adjuvants (buffering agents, dispersing agent) can also be incorporated into the pharmaceutical composition. Carriers can contain a solution of the imaging agent or a cocktail thereof dissolved in an acceptable carrier, preferably an aqueous carrier. A variety of aqueous sterile carriers can be used, e.g., water, buffered water, 0.4% saline, 0.3% glycine and the like. The solutions should be pyrogen-free, sterile, and generally free of particulate matter.

The compositions can contain additional pharmaceutically acceptable substances as necessary to approximate physiological conditions such as pH adjusting and buffering agents, toxicity adjusting agents and the like, for example sodium acetate, sodium chloride, potassium chloride, calcium chloride, and sodium lactate.

The concentration of imaging agent in the composition solutions may vary as required. Typically, the concentration will be in trace amounts of about 10⁻⁷% to about 10⁻⁴% by weight to as much as about 5% by weight depending on the imaging modality, and are selected primarily based on fluid volumes and viscosities in accordance with the particular mode of administration selected.

A typical composition for intravenous infusion can be made to contain 250 ml of sterile Ringer's solution and up to 1 mg of the imaging agent. The composition containing the imaging agent can be administered subcutaneously, intramuscularly or intravenously to patients.

In one embodiment the pharmaceutical carrier can be any compatible, non-toxic substance suitable for delivery of a labeled beta-amyloid ("A-beta") binding compound to the patient. Such carriers and compounds are disclosed in U.S. Patent Application Serial No. 101431,202. Such compounds can include imaging agents that bind to soluble beta-amyloid such as small molecules, antibodies, antibody fragments, nucleic acids, proteins, peptides, dendrimers, and polymers. Forms of beta-amyloid to which these compounds bind includes monomers, dimers, trimers and oligomers of A-beta 1-38, A-beta 1-39, A-beta 1-40, A-beta 1-41, A-beta 1-42, A-beta 1-43 or any combination thereof. Suitable carriers include sterile water, alcohol, fats, waxes, proteins, and inert solids may be included in the carrier. Pharmaceutically acceptable adjuvants (buffering agents, dispersing agent) can also be incorporated into the pharmaceutical composition.

To obtain an image, the imaging agent is administered to a subject. After administration, clearance time can, if desired, be permitted which allows the imaging agent to travel throughout the subject's body and accumulate in a manner dictated by the targeting moiety selected, whereas the unbound imaging agent passes through the subject's body. The clearance time will vary depending on the detectable species chosen for use and can range from 1 minute to 24 hours. The imaging agent is then detected noninvasively in the subject's body by positron emission tomography ("PET"). Equipment and methods for the PET imaging are readily available and well known to those skilled in the art.

The imaging agent produced using compounds in accordance with this disclosure can be used, for example, to diagnose or assess disease or pre-disease states. The present compounds also can be used to determine the efficacy of therapies. Using multiple images over time, physicians can determine the amount and frequency of therapy needed by an individual subject. In this embodiment, an imaging agent in accordance with the present disclosure is administered and a baseline image is obtained. Then, the therapy to be evaluated is administered to the subject. After a pre-determined period of time, a second administration of an imaging agent in accordance with the present disclosure is given. A second image is obtained. By qualitatively and quantitatively comparing the baseline and the second image, the effectiveness of the therapy being evaluated can be determined based on a decrease of the signal intensity of the second image. It should, of course, be understood that the treatment being evaluated can be administered before the first dose of the imaging agent, if desired.

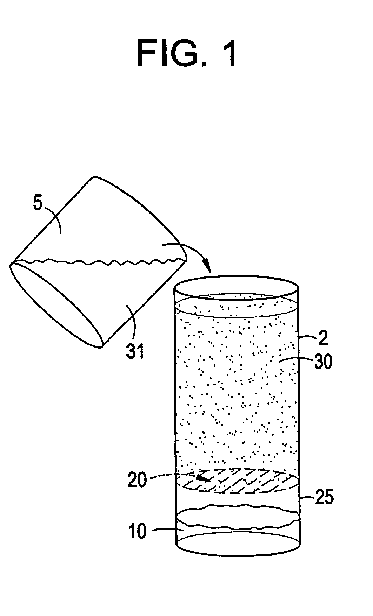

In one embodiment shown in Figure 1, a kit in accordance with this disclosure includes a first container 5 that hold a solution 31 that contains the detectable species used to form the imaging agent in accordance with the methods described hereinabove. The kit also includes a second container 2 that holds particles of the solid support material 30 that has bound thereto a compound in accordance with this disclosure that includes both an imaging moiety and a regioselective leaving group.

Container 2 includes a permeable membrane 20 at the lower end thereof to prevent passage of particulate material 30 out of container 2.

To prepare the imaging agent, solution 31 is poured out of container 5 into container 2. As solution 31 passes through particulate material 30, a substitution reaction takes place to provide an injectable solution 10 containing the imaging agent that includes the targeting moiety and the detectable species. The injectable solution 10 passes through membrane 20 into collection vessel 25. Any by-product from the leaving group remains bound to the solid particulate 30 and is retained within container 2 by membrane 20. Injectable solution 10 can then be drawn into a conventional syringe (not shown) and administered to a patient.

In particularly useful embodiments, container 2 is dimensioned and configured to fit into a microwave accelerated chemistry set such as those commercially available from CEM Corp. (Matthews, NC) or Personal Chemistry (Uppsala, Sweden). In this embodiment, the reactants can be subjected to microwave promoted chemistry as described in Lidstrom et al., "Microwave assisted organic synthesis - a review", Tetrahedron 2001, 57, 9225.

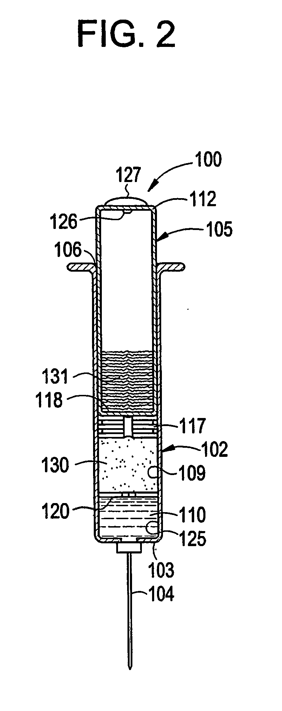

In an alternative embodiment shown in Figure 2, the components of the kit are assembled to form a disposable syringe, generally indicated as reference 100. Thus the kit includes a cylindrical plastic tube or cylinder 102 whose forward end 103 is narrowed at its outlet including attaching means for fitting a detachable hollow needle 104 to be used for injecting, for example, compositions containing the imaging agent into a patient's body. Prior to being used, this needle attaching means is normally capped with a rubber plug (not shown).

This hollow cylindrical tube 102 includes rear opening 106 through which the second component of the kit, namely cylindrical hollow piston 105 is inserted. As illustrated in Figure 2, before use part of said piston 105 projects from the tube 102.

Into said piston 105 a predetermined amount of a solution containing the detectable solution 131 is charged (for example, a solution containing H¹⁸F). Tube 102 contains a solid particulate 130 in accordance with one embodiment of this disclosure in the form of a solid support having bound thereto a compound that includes a targeting moiety and a regioselective leaving group (see Figure 2). Permeable membrane 120 positioned below the particulate component 130 defines a space 125 capable of containing the injectable solution 110 that includes the imaging agent which is the reaction product of solution 131 and particulate component 130. Membrane 120 has openings sufficiently small to prevent passage of particulate component 130, but allows passage of the injectable liquid composition 110 containing the imaging agent.

Hollow cylindrical piston 105 includes a fluid orifice 118 and coupling projecting plug 117. The structure and operation of coupling plug 117 are known and described in detail in U.S. Patent No. 6,379,328.

Hollow piston 105 can be pre-filled with the solution containing the detectable species. To this end, piston 105 includes at its rear end 112 an orifice 126 by which the solution is injected by conventional means. Orifice 126 can be closed with a rubber plug 127. This plug is tightly fitted so as to maintain a total sterilization of solution 131 inside said piston 105. For safety purposes, plug 127 may be sealed by the manufacturer.

In order to mix the solution 131 and the particulate component 130 so that the reaction yielding the imaging agent can be formed, piston 105 is rotated as described in detail in U.S. Patent No. 6,379,328 until coupling plug 117 forms a fluid passage and there is a fluid communication between piston 105 and tube 102. Once the fluid passageway is formed, the user pulls the upper half of piston up thereby creating a vacuum effect inside inner volume 109. Due to this vacuum effect solution 131 contained inside piston 105 enters into the inner volume or chamber 109 providing an opportunity for the required substitution reaction to occur between said solution 131 and solid particulate component 130 already lodged in said inner volume 109.

In order to carry out the injection operation, piston 105 must be rotated again in order to close the fluid passageway, and advanced thus impelling fluid first into space 125 and ultimately outwards through the hollow needle 104.

In the embodiment as illustrated in Figure 2, once the injection operation is finished, piston 105 is completely lodged in tube 102. Thus, the syringe in this embodiment can not be reused.

As in the previously described embodiment, the entire assembly described above may be tailored such as to fit into a microwave resonant cavity, thus allowing for microwave accelerated synthesis to occur. As described in Lidstrom et al. ("Microwave assisted organic synthesis - a review", Tetrahedron 2001, 57, 9225) shorter reaction times and sometimes better yields result from the application of this methodology.

Imaging agents prepared according to the invention may be administered to a patient or subject. The method includes forming a compound that includes a targeting moiety bound to a leaving group that contains a site for regioselective substitution of a detectable species. The compound is contacted with a solution containing the detectable species to form a reaction mixture, the detectable species is recovered, and the detectable species is then administered to a subject, before detecting the detectable species and generating an image.

### EXAMPLES

In the following examples, a benzyl or 3,4,5-trimethoxy benzyl moiety was utilized as the imaging agent. Thus, where ¹⁸F was used as a labeling radioisotope, the kinetics of the labeling process was based on the amount of benzyl fluoride (or 3,4,5-trimethoxybenzyl fluoride) released over time, upon treatment with KF-K2.2.2 complex (utilizing 'cold' KF, i.e. with ¹⁹F). Similarly, where ¹²⁴I was used as radioisotope, the formation of the corresponding benzyl iodide (with ordinary K¹²⁷I) was monitored vs. time. Finally, where "C was the desired radioisotope, the formation of ethylbenzene or 1-ethyl-3,4,5-trimethoxy benzene was monitored upon reaction with MeLi (¹²CH₃Li, commercially available from Aldrich). In order to evaluate the chemistry, naturally abundant (non-radioactive) isotopes, sometimes referred to as 'cold' compounds, were used because they do not produce radiation.

The radioactive isotope was substituted for the naturally abundant one (e.g., the chemistry is developed with ¹⁹F, but imaging was done with ¹⁸F). All of the 'cold labeled' products were identified and quantified by GC-MS.

In each of the following examples, reaction progress was monitored by GC-MS analysis (Hewlett-Packard 5890 series II, DB-5 MS column, temperature gradient) following the manufacturer's instructions and using software provided by the manufacturer. Purifications were done by medium pressure flash chromatography (MPFC) using an ISCO CombiFlash Companion chromatograph and solvent gradient. Microwave accelerated synthesis was conducted using a CEM Explorer microwave synthesis station.

### Example 1

2-Mercapto-1-methylimidazole (228.5 mg, 2 mmol), N,N-diisopropylethylamine (0.45 ml, 1.25 eq.) and 3,4,5-trimethoxybenzyl chloride (455 mg, 1.05 eq.) were added to 2 ml dry dimethylformamide and the mixture was stirred at room temperature for 4 hours. The solvent was removed under reduced pressure and the residue was purified by MPFC (hexane/dichloromethane gradient) to give the desired 1-methyl-2-(3,4,5-trimethoxybenzylthio)-imidazole as a waxy, white solid in 82% yield, which was identified as compound 1. The structure of this compound was as follows:

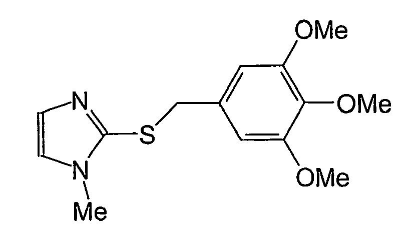

### Example 2

5-Methoxy-2-benzimidazolethiol (181 mg, 1 mmol), N,N-diisopropylethylamine (0.22 ml, 1.25 eq.) and benzyl bromide (130 µl, 1 eq.) were mixed in 1 ml dry dichloromethane and stirred at room temperature for 3 hrs. The crude product was purified by MPFC (hexanes-ethyl acetate 10-75% v/v) to give the desired 5-methoxy-2-benzylthio-benzimidazole as white crystals in 68% yield, which was identified as compound 2. The structure of this compound was as follows:

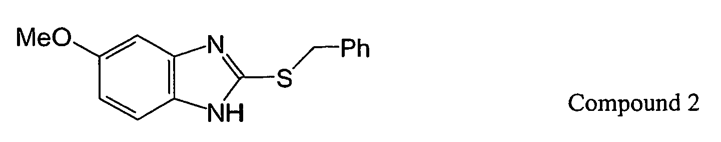

### Example 3

Following the procedure of Example 2, 1-methyl-2-benzylthio-pyrimidine was prepared from benzyl bromide and 2-mercapto-1-methylpyrimidine in 85% yield, which was identified as compound 3. The structure of this compound was as follows:

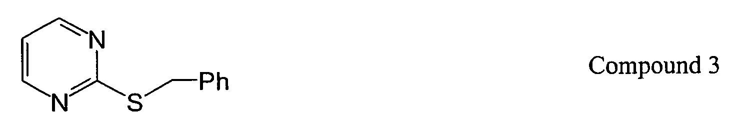

### Example 4

To a dry vial was added compound 2 (26.4 mg, 98 µmol), dry tetrahydrofuran (0.2 ml) dry N,N-diisopropylethylamine (18µl, 1.5 eq.), followed by dropwise addition of freshly distilled methyl triflate (11.5 µl, 1.08 eq.). The colorless solution was stirred at room temperature overnight and the desired product was purified by MPFC (hexanes-ethylacetate 10-60% v/v) to give 1-methyl-5-methoxy 2-benzylthio benzimidazole and 1-methyl-6-methoxy-2-methylthio benzimidazole (62%, combined mixture of the two regioisomers), which were identified as compounds 4a and 4b. (Compound 2 was merely an intermediate necessary in the synthesis of compounds 4a and 4b.) The structures of these compounds were as follows:

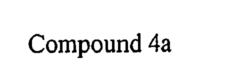

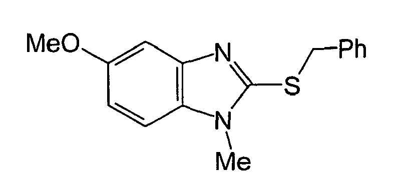

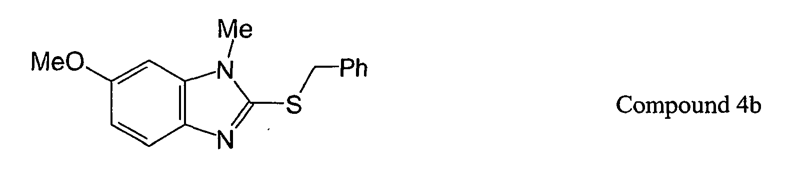

### Example 5

The following is a general procedure for the in-situ formation of the onium salts, followed by simulated labeling (addition of the nucleophile).

To a dry vial was added the imidazole, pyrimidine or benzimidazole precursor (0.1 mmol) and dry dichloromethane (0.2 ml). The vial was capped, and methyl triflate was then added dropwise (12µl, 1.05 eq.) and the vial was stirred at room temperature overnight. The onium salt was generally soluble under these conditions. A solution of the nucleophile, 2 equivalents, was then added, followed by 10µl of a 1M solution of 3,4-dimethoxytoluene (0.01 mmol) in dry THF as internal standard. The vial was placed in a microwave tube and irradiated under continuous cooling for 5 - 15 minutes at a preselected temperature of 60°C. Upon completion of the procedure, samples were removed and analyzed by GC-MS. The following solutions were used as nucleophiles: KX-K222 complex (X=F, Br, I] in dry acetonitrile (0.1 M) for simulating ¹⁸F, ⁷⁶Br and ¹²⁴I labeling. MeLi (3.0 M in THF) for ¹¹C labeling. The onium salt was prepared substituting tetrahydrofuran for dichloromethane in this latter case. A white microcrystalline precipitate formed, but the reaction proceeded well upon the addition of the methyllithium. The structures for the onium salts prepared from compounds 1, 3, 4a, and 4b (in each case, the counterion was triflate, CF₃SO₃⁻) were as follows:

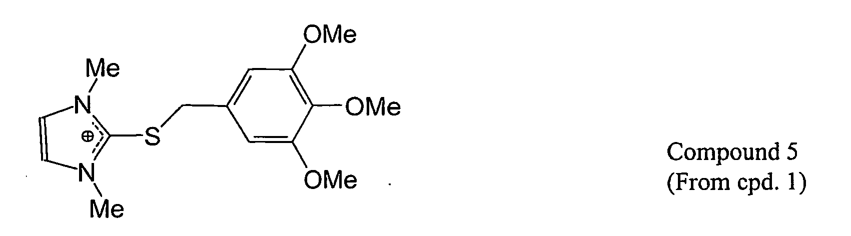

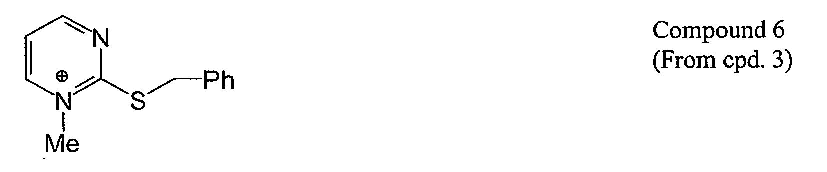

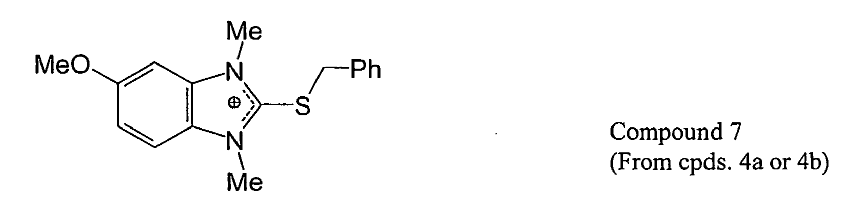

What follows below in Table 1 is a summary of the reaction conditions leading to the formation of the various onium salts depicted above.

**Table 1**

| Onium salt | Nucleophile | time | yield of | Note |
|---|---|---|---|---|
| 'labeled' | | | | |
| | | | product | |
| Compound 6 | F- | 5min | 2% | 60°C |
| Compound 6 | F- | 20 min | 7% | 80°C |
| Compound 7 | Br⁻ | 5min | 54% | 60°C |
| | I⁻ | 5min | 78% | 60°C |
| | CH₃⁻ | 5min | 95% | 60°C |
| | | | | |
| Compound 5 | Br⁻ | 5min | 43% | 60°C |
| | I⁻ | 5min | 69% | 60°C |
| | CH₃⁻ | 5min | 87% | 60°C |

In the above Table, "Time" refers to the total microwave irradiation time, during which air cooling was also begun. That is, once the sample was introduced into the microwave cavity, both microwave power and air cooling were switched on simultaneously and left on for the entire length of the microwave accelerated reaction. The equipment permitted the setting of a temperature limit for the experiment, which was automatically maintained by adjusting the total microwave power delivered. An infrared sensor read the bottom temperature of the vial, recording the data and providing feedback for the temperature regulation. While temperature excursions occurred at the small reaction scale used, they were generally limited to ∼ 10°C by setting a maximum power ceiling.

As can be seen from Table 1, with the exception of onium salt compound 6 (prepared from compound 3), reaction with fluoride did not proceed appreciably for the remaining onium salts under conditions that would be compatible with the short lived radioisotope ¹⁸F (i.e., no longer than 1 half-life, 110 min.).

## Claims

1. A compound comprising a targeting moiety bound to a leaving group, wherein the leaving group is selected from :
(i) groups of the formula: 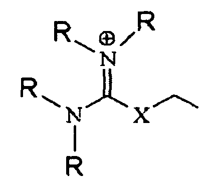 where X is S, O and R can be the same or different at each occurrence and is selected from C₁ to C₂₀ alkyl groups;
(ii) groups of the formula: 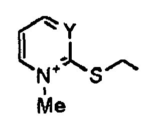 where Y is N or CH;
(iii) groups of the formula: 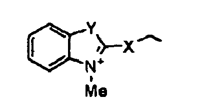 where when X is S, then Y is O or S and where when X is O, then Y is S;
(iv) groups of the formula: 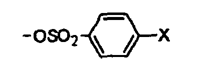 where X is selected from C₄ to C₁₀ alkylene, -CN, -N⁺(CH₃)₃, or -(Q)ₙOCH₃ where Q is C₂ to C₆ alkoxy and n =1 to 6;
(v) groups of the formula: 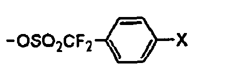 where X is selected from C₄ to C₁₀ alkylene, -CN, -N⁺(CH₃)₃, or-(Q)ₙOCH₃ where Q is C₂ to C₆ alkoxy and n = 1 to 6; and
(vi) groups of the formula:
-OSO_{y}CF₂·CH₂X
where X is selected from C₄ to C₁₀ alkylene, -CN, -N⁺(CH₃)₃, or -(Q)ₙOCH₃ where Q is C₂ to C₆ alkoxy and n = 1 to 6.

2. A compound as in claim 1 wherein the targeting moiety is selected from proteins, glycoproteins, lectins, peptides, polypeptides, saccharides, vitamins, steroids, steroid analogs, hormones, cofactors, nucleosides, nucleotides and polynucleotides.

3. A method of producing an imaging agent comprising the steps of:
providing a compound according to claim 1;
contacting the compound with a solution containing the detectable species to form a reaction mixture; and
recovering the imaging agent.

4. A method as in claim 3 wherein the step of contacting the compound with a solution containing the detectable species comprises contacting the compound with a solution containing ¹⁸F.

5. A kit comprising:
a first container (5) having therein a solution (31) containing a detectable species; and
a second container (2) having therein a compound that includes a targeting moiety bound to a support (30) via a leaving group according to claim 1 that contains a site for regioselective substitution of the detectable species.

## Patentansprüche

1. Verbindung, umfassend eine an eine Abgangsgruppe gebundene zielsteuernde Einheit, wobei die Abgangsgruppe ausgewählt ist aus:
(i) Gruppen der Formel: 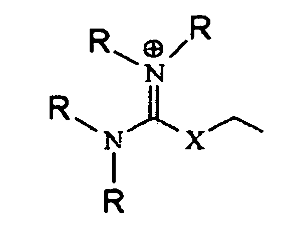 wobei X für S, O steht und R bei jedem Auftreten gleich oder unterschiedlich sein kann und aus C₁- bis C₂₀-Alkylgruppen ausgewählt ist;
(ii) Gruppen der Formel: 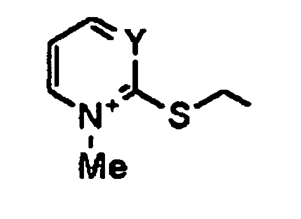 wobei Y für N oder CH steht;
(iii) Gruppen der Formel: 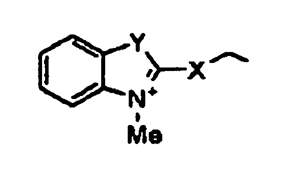 wobei, wenn X für S steht, Y für O oder S steht und wobei, wenn X für O steht, Y für S steht;
(iv) Gruppen der Formel: 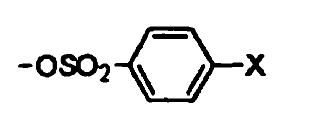 wobei X ausgewählt ist aus C₄- bis C₁₀-Alkylen, -CN, -N⁺(CH₃)₃ oder - (Q)ₙOCH₃, wobei Q für C₂- bis C₆-Alkoxy steht und n = 1 bis 6 ist;
(v) Gruppen der Formel: 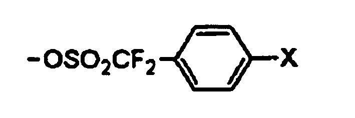 wobei X ausgewählt ist aus C₄- bis C₁₀-Alkylen, -CN, -N⁺(CH₃)₃ oder - (Q)ₙOCH₃, wobei Q für C₂- bis C₆-Alkoxy steht und n = 1 bis 6 ist; und
(vi) Gruppen der Formel:
**- OSO₂CF₂·CH₂X**
wobei X ausgewählt ist aus C₄- bis C₁₀-Alkylen, -CN, -N⁺(CH₃)₃ oder - (Q)ₙOCH₃, wobei Q für C₂- bis C₆-Alkoxy steht und n = 1 bis 6 ist.

2. Verbindung nach Anspruch 1, wobei die zielsteuernde Einheit ausgewählt ist aus Proteinen, Glykoproteinen, Lektinen, Peptiden, Polypeptiden, Sacchariden, Vitaminen, Steroiden, Steroidanalogen, Hormonen, Cofaktoren, Nukleosiden, Nukleotiden und Polynukleotiden.

3. Verfahren zur Herstellung eines Bildgebungsmittels, umfassend die Schritte:
Bereitstellen einer Verbindung nach Anspruch 1;
In-Kontakt-Bringen der Verbindung mit einer die nachweisbare Spezies enthaltenden Lösung, um ein Reaktionsgemisch zu bilden; und
Wiedergewinnen des Bildgebungsmittels.

4. Verfahren nach Anspruch 3, wobei der Schritt des In-Kontakt-Bringens der Verbindung mit einer Lösung, welche die nachweisbare Spezies enthält, das In-Kontakt-Bringen der Verbindung mit einer Lösung umfasst, welche ¹⁸F enthält.

5. Kit, umfassend:
einen ersten Behälter (5), der in demselben eine Lösung (31) aufweist, die eine nachweisbare Spezies enthält; und
einen zweiten Behälter (2), der in demselben eine Verbindung aufweist, die eine zielsteuernde Einheit beinhaltet, die an einen Träger (30) mittels einer Abgangsgruppe nach Anspruch 1 gebunden ist, die eine Stelle für eine regioselektive Substitution der nachweisbaren Spezies enthält.

## Revendications

1. Composé comprenant une fraction de ciblage liée à un groupe partant, dans lequel le groupe partant est sélectionné à partir de :
(i) groupes selon la formule : 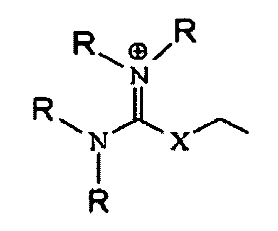 dans laquelle X représente S, O, et R peut être identique ou différent à chaque occurrence et est sélectionné à partir de groupes alkyles en C₁ à C₂₀ ;
(ii) groupes selon la formule : 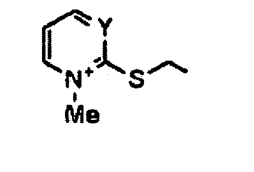 dans laquelle Y représente N ou CH ;
(iii) groupes selon la formule : 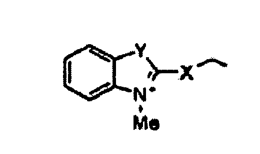 dans laquelle, lorsque X représente S, alors Y représente O ou S et dans laquelle, lorsque X représente O, alors Y représente S ;
(iv) groupes selon la formule : 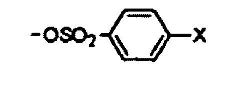 dans laquelle X est sélectionné à partir d'un alkylène en C₄ à C₁₀, de -CN, - N⁺(CH₃)₃, ou de -(Q)ₙOCH₃ dans lequel Q est un alkoxy en C₂ à C₆ et n = 1 à 6;
(v) groupes selon la formule : 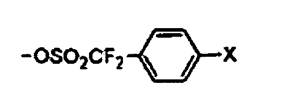 dans laquelle X est sélectionné à partir d'un alkylène en C₄ à C₁₀, de -CN, - N⁺(CH₃)₃, ou de -(Q)ₙOCH₃ dans lequel Q est un alkoxy en C₂ à C₆ et n = 1 à 6; et
(vi) groupes selon la formule :
-OSO₂CF₂CH₂X
dans laquelle X est sélectionné à partir d'un alkylène en C₄ à C₁₀, de -CN, - N⁺(CH₃)₃, ou de -(Q)ₙOCH₃ dans lequel Q est un alkoxy en C₂ à C₆ et n = 1 à 6.

2. Composé selon la revendication 1, dans lequel la fraction de ciblage est sélectionnée à partir de protéines, de glycoprotéines, de lectines, de peptides, de polypeptides, de saccharides, de vitamines, de stéroïdes, d'analogues de stéroïde, d'hormones, de cofacteurs, de nucléosides, de nucléotides et de polynucléotides.

3. Procédé de production d'un agent d'imagerie comprenant les étapes de :
production d'un composé selon la revendication 1 ;
mise en contact du composé avec une solution contenant l'espèce détectable afin de former un mélange de réaction ; et
récupération de l'agent d'imagerie.

4. Procédé selon la revendication 3, dans lequel l'étape de mise en contact du composé avec une solution contenant l'espèce détectable comprend la mise en contact du composé avec une solution contenant du 18^{F}.

5. Kit comprenant :
un premier conteneur (5) comportant, à l'intérieur, une solution (31) contenant une espèce détectable ; et
un second conteneur (2) comportant, à l'intérieur, un composé qui comprend une fraction de ciblage liée sur un support (30) par l'intermédiaire d'un groupe partant selon la revendication 1, qui contient un site afin d'assurer une substitution régiosélective de l'espèce détectable.
